(19) **Europäisches Patentamt**
**European Patent Office**
**Office européen des brevets**

(11) **EP 0 797 540 B2**

(12) **NOUVEAU FASCICULE DE BREVET EUROPEEN**
Après la procédure d'opposition

(45) Date de publication et mention de la décision concernant l'opposition:
**03.12.2008 Bulletin 2008/49**

(45) Mention de la délivrance du brevet:
**21.04.1999 Bulletin 1999/16**

(21) Numéro de dépôt: **95905678.9**

(22) Date de dépôt: **28.12.1994**

(51) Int Cl.:
***C01B 33/193*** (2006.01)

(86) Numéro de dépôt international:
**PCT/FR1994/001543**

(87) Numéro de publication internationale:
**WO 1995/018066 (06.07.1995 Gazette 1995/29)**

(54) **SILICES ABRASIVES POUR COMPOSITIONS DENTIFRICES**

SCHLEIFMITTELKIESELSÄURE FÜR ZAHNPASTAZUSAMMENSETZUNGEN

ABRASIVE SILICAS FOR TOOTHPASTE COMPOSITIONS

(84) Etats contractants désignés:
**CH DE ES FR GB IT LI NL**

(30) Priorité: **29.12.1993 FR 9315824**

(43) Date de publication de la demande:
**01.10.1997 Bulletin 1997/40**

(73) Titulaire: **RHODIA CHIMIE**
**92408 Courbevoie Cédex (FR)**

(72) Inventeurs:
• **CHEVALLIER, Yvonick**
**F-69270 Fontaines-Saint-Martin (FR)**
• **DROMARD, Adrien**
**F-69006 Lyon (FR)**

(74) Mandataire: **Delenne, Marc et al**
**Rhodia Services,**
**Direction de la Propriété Industrielle,**
**40, rue de la Haie-Coq**
**93306 Aubervilliers Cedex (FR)**

(56) Documents cités:
| | |
|---|---|
| **EP-A- 0 139 754** | **EP-A- 0 236 070** |
| **EP-A- 0 396 460** | **EP-A- 0 535 943** |
| **EP-A1- 0 396 459** | **EP-A1- 0 643 015** |
| **US-A- 4 243 428** | |

EP 0 797 540 B2

**Description**

La présente invention concerne l'utilisation de silices, en particulier de silices précipitées, comme agents abrasifs dans des compositions dentifrices.

De très nombreuses compositions dentifrices ont été développées et commercialisées depuis plusieurs années.

On sait que les formulations dentifrices peuvent contenir divers composants, notamment de l'eau, un humectant (par exemple de la glycérine, du sorbitol, du xylitol ou du polyéthylène glycol...), un agent épaississant (par exemple de la gomme xanthane), une source de fluorure (le plus souvent le fluorure de sodium ou le monofluorophosphate de sodium (agent anti-carrie)), un agent de coloration, un arôme, un agent sucrant, un parfum, un agent conservateur, un tensio-actif et/ou un additif thérapeutique ...

Elles contiennent généralement aussi un agent abrasif qui doit permettre par son action mécanique l'élimination de la plaque dentaire, tout en ne soumettant pas les dents elles-mêmes à une abrasion inacceptable.

Parmi les agents abrasifs habituellement employés, on peut citer les phosphates et carbonates de calcium, les métaphosphates de sodium, les alumines et, depuis quelques années, les silices.

Néanmoins, les silices de l'art antérieur utilisées comme agents abrasifs dans les compositions dentifrices ne présentent pas toujours l'indice de réfraction et la porosité souhaitables.

La demande EP-A-139754 vise une silice pour dentifrice, présentant une surface BET et une surface spécifique CTAB de 5-60 $m^2$/g-anhydride, avec une différence de moins de 40 $m^2$/g-anhydride entre la valeur BET et la valeur CTAB, et un indice de réfraction de 1,42-1,45. Cette silice est obtenue par réaction en deux étapes, d'une solution d'un silicate de métal alcalin et d'acide chlorhydrique ou sulfurique, en présence d'un électrolyte ; la première étape, de cristallisation, est réalisée jusqu'à un pH de 10,0 , et celle de neutralisation jusqu'à pH 8,0-6,5, puis mûrissement pendant au moins 10 minutes ; le rapport des vitesses d'introduction de l'acide entre les deux étapes est d'au moins 5/3.

La demande de brevet EP-A-236070 vise une silice abrasive pour dentifrice, présentant une surface spécifique de 10 à 450 $m^2$/g, un diamètre de particule de 3 à 20 pm, une abrasion perspex de 23 à 35 et éventuellement une absorption d'huile (huile de lin) de 60-100 cc/100 g. Celle-ci peut être obtenue par précipitation, par réaction d'un silicate de métal alcalin et d'un acide minéral à un pH de 10-10,5 en présence d'un électrolyte.

La présente invention est relative à l'utilisation dans des compositions dentifrices de silices à basse surface spécifique, présentant à la fois un pouvoir abrasif élevé, un indice de réfraction et une prise d'huile (ou absorption d'huile) relativement bas, ces silices étant de plus compatibles avec les composés organiques aminés.

Ainsi, l'objet de l'invention est l'utilisation d'une silice, de préférence une silice précipitée, comme agent abrasif dans une composition dentifrice comprenant un composé organique aminé présentant :

   i) une surface spécifique BET comprise entre 20 et 75 $m^2$/g,
   ii) une surface spécifique CTAB comprise entre 16 et 45 $m^2$/g,
   iii) une abrasivité RDA comprise entre 120 et 160,
   iv) un indice de réfraction compris entre 1,435 et 1,450,
   v) une prise d'huile DOP comprise entre 70 et 105 ml/100g, et
   vi) une compatibilité avec les composés organiques aminés, notamment avec les amines fluorées, d'au moins 50 %.

Dans le présent exposé, la surface spécifique BET est déterminée selon la méthode de BRUNAUER-EMMET-TELLER décrite dans "The Journal of the American Chemical Society", vol. 60, page 309, février 1938 et correspondant à la norme ISO 5794/1 (annexe D).

La surface spécifique CTAB est la surface exteme déterminée selon la norme NFT 45-007 (novembre 1987) (5.12).

L'abrasivité RDA ("Radioactive Dentine Abrasion") est mesurée selon la méthode décrite par J.J. HEFFERREN dans "Journal of Dental Research", vol. 55(4), page 563, 1976.

Selon cette méthode, des dents humaines irradiées par un flux de neutrons sont soumises à un certain brossage mécanique ; l'indice d'abrasion du dentifrice testé correspond à la radioactivité $^{32}$P émanant de la dentine. On choisit comme référence une suspension contenant 10 grammes de pyrophosphate de calcium dans 50 ml d'une solution aqueuse à 0,5 % de cellulose de carboxyméthyle de sodium, référence dont la RDA est fixée arbitrairement à 100. La silice dont on désire déterminer la RDA est mise en suspension comme le pyrophosphate de calcium et soumise au même brossage mécanique.

L'indice de réfraction de la silice dans le sorbitol est celui de la suspension la plus transparente (donc transmission maximum) de cette silice dans diverses solutions eau-sorbitol, transparence déterminée par la transmission à 589 nm avec un spectrophotomètre. Chaque suspension est obtenue par dispersion d'1 gramme de silice dans 19 grammes de solution eau-sorbitol, puis désaération sous léger vide avant lecture de la transmission (lecture faite avec, comme produit de référence, la solution eau-sorbitol sans silice) sur le spectrophotomètre et de l'indice de réfraction sur un réfractomètre.

La prise d'huile DOP est déterminée selon la norme ISO 787/5 en mettant en oeuvre le dioctylphtalate.

La taille moyenne en poids $D_{50}$ des particules de la silice est déterminée à l'aide d'un appareil SYMPATEC HELOS. Cet appareil applique le principe de la diffraction FRAUNHÖFFER et met en oeuvre un laser He/Ne de faible puissance. L'échantillon est préalablement dispersé par application d'ultra-sons dans l'eau pendant 30 secondes pour obtenir une suspension aqueuse.

Le pH de la silice est mesuré selon la norme ISO 787/9 (pH d'une suspension à 5 % dans l'eau).

La compatibilité de la silice avec le dihydrofluorure de bis(hydroxyéthyl)aminopropyl-N-(hydroxyéthyl-octadécylamine), qui est une amine fluorée et donc un composé organique aminé, est déterminée de la manière suivante :

- une solution standard contenant 1,65 % d'amine fluorée est préparée en ajoutant 5 g de solution commerciale d'amine fluorée à 33 % dans le propanediol à 95 g d'eau bidistillée ;
- une suspension aqueuse (ou slurry) de silice est formée par dispersion de 6 g de silice dans 24 g de la solution standard préparée précédemment, puis est maintenue sous agitation pendant 24 heures à 37 °C après ajustement de son pH à 5,0 (par addition d'acide chlorhydrique 2N) ;
- cette suspension est ensuite centrifugée à 10000 t/mn pendant 30 minutes et le surnageant obtenu donne, après filtration sur filtre Millipore 0,22 $\mu$m, une solution dite solution de mesure ;
- la concentration en amine fluorée des solutions standard et de mesure est déterminée par turbidimétrie, en l'occurrence en mesurant avec une phototrode calée sur 550 nm la turbidité résultant de la formation de miscelles entre l'amine fluorée et un composé anionique (l'Aerosol OT, constitué par du dioctylsulfosuccinate de sodium) ;
- la compatibilité en amine fluorée (compatibilité AF) de la silice est donnée par le rapport

$$\frac{\text{concentration en amine fluorée dans la solution de mesure}}{\text{concentration en amine fluorée dans la solution standard}}$$

Les silices utilisée selon l'invention présentent tout d'abord de faibles surfaces spécifiques.

Leur surface spécifique BET est comprise entre 20 et 75 m$^2$/g, de préférence entre 35 et 64 m$^2$/g et, par exemple, entre 45 et 59 m$^2$/g ; leur surface spécifique CTAB est comprise entre 16 et 45 m$^2$/g, de préférence entre 20 et 40 m$^2$/g et, par exemple, entre 24 et 36 m$^2$/g.

En général, la différence entre la surface spécifique BET et la surface spécifique CTAB d'une même silice utilisée selon l'invention est d'au plus 35 m$^2$/g, par exemple d'au plus 25 m$^2$/g.

Une abrasivité élevée caractérise également les silices employée selon l'invention : celles-ci possèdent une abrasivité RDA comprise entre 120 et 160, notamment entre 125 et 145.

L'indice de réfraction desdites silices est peu élevé : il est ainsi compris entre 1,435 et 1,450, de préférence entre 1,438 et 1,446, par exemple entre 1,440 et 1,444. Elles présentent alors généralement une transmission supérieure à 70 %, de préférence supérieure à 75 %, voire supérieure à 80 %.

Les silices utilisée selon l'invention possèdent aussi une prise d'huile DOP assez basse : celle-ci est comprise entre 70 et 105 ml/100g, de préférence entre 80 et 105 ml/100g et, plus particulièrement, entre 85 et 95 ml/100g.

Elles sont compatibles avec les composés organiques aminés, qui sont souvent présents dans les formulations de dentifrice. Par "composé organique aminé", on entend toute molécule active intervenant dans les formulations de dentifrice et contenant au moins un atome d'azote ; on peut citer notamment les amines fluorées, utilisées comme agents anti-carie, telles que le dihydrofluorure de bis(hydroxyéthyl)-aminopropyl-N-(hydroxyéthyl-octadécylamine).

La compatibilité des silices employée selon l'invention avec les composés organiques aminés, notamment avec les amines fluorées, définie selon le test décrt ci-dessus, est ainsi d'au moins 50 %, plus particulièrement d'au moins 55 %.

De même, les silices utilisée selon l'invention sont, en général, compatibles avec les cations métalliques, qui interviennent fréquemment dans les formulations de dentifrice, notamment avec les cations métalliques bivalents et plus, en particulier le zinc, le strontium, l'étain ; ces cations peuvent être sous forme de sels minéraux : on peut citer par exemple le citrate, sulfate ou fluorure de zinc, le chlorure de strontium, le fluorure d'étain.

Cette compatibilité desdites silices avec les composés organiques aminés et, en général, avec les cations métalliques a pour conséquence que ceux-ci peuvent remplir, au moins en grande partie, la fonction qui leur est initialement impartie, ce qui n'est souvent pas le cas avec les silices de l'art antérieur, en particulier les silices dites abrasives.

D'une manière générale, elles présentent une taille moyenne en poids $D_{50}$ des particules comprise entre 4 et 20 $\mu$m, par exemple entre 5 et 12 pm.

Le pH des silices employées selon l'invention est, généralement, compris entre 6,2 et 7,4.

La silice utilisée selon l'invention peut être obtenue par réaction d'un silicate de métal alcalin M avec un agent acidifiant ce par quoi l'on obtient une suspension de silice précipitée, puis la séparation et le séchage de cette suspension, la précipitation étant réalisée selon les étapes successives suivantes :

(i) on forme un pied de cuve initial comportant une partie de la quantité totale du silicate de métal alcalin M engagé dans la réaction et au moins un électrolyte, la concentration en silicate exprimée en $SiO_2$ dans ledit pied de cuve initial étant comprise entre 35 et 100 g/l et la concentration en électrolyte dans ledit pied de cuve initial étant comprise entre 10 et 40 g/l,

(ii) on ajoute de l'agent acidifiant audit pied de cuve initial jusqu'à ce que 50 à 85 % de la quantité de $M_2O$ présente dans ledit pied de cuve initial soient neutralisés,

(iii) on ajoute au milieu réactionnel simultanément de l'agent acidifiant et la quantité restante du silicate, le pH du milieu réactionnel étant maintenu entre 8,6 et 9,6 pendant cette étape (iii),

(iv) on arrête l'addition de silicate et on poursuit l'addition de l'agent acidifiant dans le milieu réactionnel jusqu'à l'obtention d'une valeur de pH dudit milieu comprise entre 7,0 et 8,0,

(v) on effectue ensuite un premier mûrissement du milieu réactionnel,

(vi) on ajoute au milieu réactionnel de l'agent acidifiant jusqu'à l'obtention d'une valeur de pH du milieu comprise entre 3,7 et 4,6,

(vii) on effectue enfin un second mûrissement du milieu réactionnel.

Le choix de l'agent acidifiant et du silicate se fait d'une manière bien connue en soi.

On utilise généralement comme agent acidifiant un acide minéral fort tel que l'acide sulfurique, l'acide nitrique ou l'acide chlorhydrique, ou un acide organique tel que l'acide acétique, l'acide formique ou l'acide carbonique.

On peut par ailleurs utiliser en tant que silicate toute forme courante de silicates tels que métasilicates, disilicates et avantageusement un silicate de métal alcalin M dans lequel M est le sodium ou le potassium.

De manière générale, on emploie, comme agent acidifiant, l'acide sulfurique, et, comme silicate, le silicate de sodium.

Dans le cas où l'on utilise le silicate de sodium, celui-ci présente, en général, un rapport molaire $SiO_2/Na_2O$ compris entre 2 et 4, plus particulièrement entre 3,0 et 3,8.

Plus particulièrement, la précipitation se fait d'une manière spécifique selon les étapes suivantes.

On forme tout d'abord un pied de cuve qui comprend du silicate ainsi qu'au moins un électrolyte (étape (i)). La quantité de silicate présente dans le pied de cuve initial ne représente qu'une partie de la quantité totale de silicate engagée dans la réaction.

En ce qui concerne l'électrolyte, ce terme s'entend ici dans son acceptation normale, c'est-à-dire qu'il signifie toute substance ionique ou moléculaire qui, lorsqu'elle est en solution, se décompose ou se dissocie pour former des ions ou des particules chargées. On peut citer comme électrolyte un sel du groupe des sels des métaux alcalins et alcalino-terreux, notamment le sel du métal de silicate de départ et de l'agent acidifiant, par exemple le sulfate de sodium dans le cas de la réaction d'un silicate de sodium avec l'acide sulfurique.

La concentration en silicate dans le pied de cuve initial est comprise entre 35 et 100 g de $SiO_2$ par litre. De préférence, cette concentration est comprise entre 40 et 85 g/l, par exemple entre 45 et 75 g/l.

De même, la concentration en électrolyte dans le pied de cuve initial est comprise entre 10 et 40 g/l, de préférence entre 15 et 30 g/l, par exemple entre 19 et 25 g/l.

La deuxième étape (étape (ii)) consiste à ajouter de l'agent acidifiant audit pied de cuve initial jusqu'à ce que 50 à 85 %, de préférence 55 à 80 %, de la quantité de $M_2O$ présente dans ledit pied de cuve initial soient neutralisés.

De manière préférée, dans cette deuxième étape, on ajoute l'agent acidifiant audit pied de cuve initial jusqu'à ce que 60 à 75 % de la quantité de $M_2O$ présente dans ledit pied de cuve initial soient neutralisés.

L'agent acidifiant, utilisé dans cette deuxième étape et généralement aussi dans le reste du procédé, peut être dilué ou concentré ; sa normalité peut être comprise entre 0,4 et 36 N, par exemple entre 0,6 et 1,5 N.

En particulier, dans le cas où l'agent acidifiant est l'acide sulfurique, sa concentration est de préférence comprise entre 40 et 180 g/l, par exemple entre 60 et 150 g/l.

La durée de cette deuxième étape (étape de préneutralisation) est habituellement comprise entre 4 et 15 minutes, de préférence entre 5 et 10 minutes.

Une fois qu'est atteinte la valeur souhaitée de quantité de $M_2O$ neutralisé, on procède alors à une addition simultanée (étape (iii)) d'agent acidifiant et de la quantité restante de silicate.

Pendant cette addition simultanée, le pH du milieu réactionnel est maintenu (en régulant notamment le débit d'agent acidifiant) entre 8,6 et 9,6, de préférence entre 9,0 et 9,4, généralement à une valeur sensiblement constante.

En général, le silicate de métal alcalin M ajouté lors de cette troisième étape présente une concentration exprimée en silice comprise entre 40 et 330 g/l, par exemple entre 60 et 250 g/l.

La durée de cette troisième étape (étape d'addition simultanée) est habituellement comprise entre 20 et 90 minutes, de préférence entre 40 et 75 minutes.

A l'issue de cette étape, on arrête l'addition de silicate et on poursuit l'addition de l'agent acidifiant dans le milieu réactionnel jusqu'à l'obtention d'une valeur de pH dudit milieu comprise entre 7,0 et 8,0, de préférence entre 7,3 et 7,8 (étape (iv)).

Après arrêt de l'addition d'agent acidifiant, on laisse ensuite mûrir une première fois le milieu réactionnel (étape (v)), au pH atteint à l'issue de l'étape précédente, de préférence pendant 5 à 30 minutes, par exemple pendant 10 à 20 minutes.

Ce premier mûrissement se fait généralement à chaud, de préférence à une température constante comprise entre 75 et 98 °C, et habituellement sous agitation.

Puis, à l'issue dudit mûrissement, on ajoute de nouveau au milieu réactionnel de l'agent acidifiant jusqu'à l'obtention d'une valeur de pH dudit milieu comprise entre 3,7 et 4,6, de préférence entre 3,9 et 4,5 (étape (vi)).

Après arrêt de l'addition d'agent acidifiant, on laisse ensuite mûrir une seconde fois le milieu réactionnel (étape (vii)), au pH atteint à l'issue de l'étape précédente, de préférence pendant 5 à 30 minutes, par exemple pendant 10 à 20 minutes.

Ce second mûrissement se fait généralement aussi à chaud, de préférence à une température comprise entre 75 et 98 °C, et habituellement sous agitation.

On utilise généralement le même agent acidifiant durant tout le procédé de préparation selon l'invention.

Au cours de la réaction (étapes (i) à (vii)), la température du milieu réactionnel est généralement comprise entre 75 et 98 °C, de préférence entre 85 et 95 °C ; cette température est habituellement maintenue à une valeur sensiblement constante au cours des étapes (i) à (vii).

On obtient, à l'issue des opérations qui viennent d'être décrites, une bouillie de silice qui est ensuite séparée (séparation liquide-solide). Cette séparation consiste généralement en une filtration, suivie d'un lavage si nécessaire. La filtration peut se faire selon toute méthode convenable, par exemple à l'aide d'un filtre rotatif sous vide.

La suspension de silice ainsi récupérée (gâteau de filtration) est ensuite séchée.

Ce séchage se fait préférentiellement par atomisation.

A cet effet, on peut utiliser tout type d'atomiseur convenable, notamment un atomiseur à turbines, à buses, à pression liquide ou à deux fluides ; on utilise avantageusement un atomiseur à turbines.

Il y a lieu de noter que le gâteau à sécher n'est pas toujours dans des conditions permettant une atomisation à cause de sa viscosité trop élevée.

D'une manière connue en soi, on soumet alors le gâteau à une opération de délitage. Cette opération peut se faire par passage du gâteau dans un broyeur de type colloïdal ou à bille (fluidification par action mécanique).

A l'issue du séchage, on peut procéder à une étape de broyage sur le produit récupéré pour obtenir la granulométrie désirée ; on peut notamment employer un broyeur à couteaux, à marteaux ou un broyeur à jet d'air.

La quantité de silice utilisée selon l'invention dans les compositions dentifrices, peut varier dans de larges limites ; elle est en général comprise entre 5 et 40 % en poids, par exemple entre 5 et 25 % en poids.

Les dentifrices contenant les silices employées selon l'invention présentent de manière préférée un pouvoir nettoyant très satisfaisant. De plus, l'obtention de pâtes dentifrices translucides contenant lesdites silices est possible.

Enfin, du fait d'un indice de réfraction relativement bas (combiné à une transmission assez élevée) de la silice utilisée selon l'invention, la quantité d'humectant traditionnel à incorporer dans une composition dentifrice contenant ladite silice peut être réduite et remplacée en partie par de l'eau, d'où une diminution du coût du produit final.

Les exemples suivants illustrent l'invention sans toutefois en limiter la portée.

EXEMPLE 1

Dans un réacteur en acier inoxydable, d'un volume de 2000 litres, muni d'un système d'agitation par hélices, d'un système d'introduction des réactifs et d'un système de chauffage par double enveloppe, on introduit:

- 117 litres d'une solution de silicate de sodium de rapport molaire $SiO_2/Na_2O$ égal à 3,6 ayant une concentration exprimée en silice de 136 g/l et étant à une température de 65 °C,
- 80 litres d'une solution aqueuse contenant 4,0 kg de $Na_2SO_4$ (électrolyte).

La concentration en silicate exprimée en $SiO_2$ et celle en électrolyte dans le pied de cuve initial sont donc respectivement de 80,7 g/l et de 20,3 g/l. Le mélange obtenu est porté à une température de 92 °C tout en le maintenant sous agitation. L'ensemble de la réaction est effectué à 92 °C.

Dans le pied de cuve ainsi formé et maintenu sous agitation, on introduit d'abord, pendant 8 mn, une solution d'acide sulfurique, de concentration égale à 80 g/l, à un débit de 7,6 l/mn ; à l'issue de cette addition, le taux de neutralisation du pied de cuve est de 67 %, c'est-à-dire que 67 % de la quantité de $Na_2O$ présente dans le pied de cuve initial sont neutralisés.

On introduit ensuite simultanément, pendant 60 mn, dans le milieu réactionnel :

- une solution de silicate de sodium telle que décrite ci-dessus (concentration exprimée en silice de 136 g/l), à une débit de 12 l/mn, et

- une solution d'acide sulfurique telle que décrite ci-dessus (concentration de 80 g/l) à un débit régulé tel que le pH du milieu réactionnel soit égal à 9,2 pendant cette addition simultanée.

On arrête ensuite l'introduction de la solution de silicate de sodium mais on poursuit l'addition de la solution d'acide sulfurique, à un débit de 7,6 l/mn, jusqu'à ce que le pH du milieu réactionnel soit égal à 7,5.

On arrête alors l'introduction de la solution d'acide sulfurique et on laisse mûrir le milieu réactionnel pendant 15 mn, au pH de 7,5 (sous agitation, à 92 °C).

On introduit ensuite de nouveau une solution d'acide sulfurique telle que décrite ci-dessus (concentration de 80 g/l), à un débit de 7,6 l/mn, jusqu'à ce que le pH du milieu réactionnel soit égal à 4,2.

On arrête alors l'introduction de la solution d'acide sulfurique et on laisse mûrir le milieu réactionnel pendant 15 mn, au pH de 4,2 (sous agitation, à 92 °C).

On obtient ainsi une bouillie de silice précipitée qui est filtrée et lavée au moyen d'un filtre rotatif sous vide.

Le gâteau de silice obtenu est ensuite fluidifié par simple action mécanique. Après cette opération de délitage, la bouillie résultante est atomisée au moyen d'un atomiseur à turbines ; le produit séché est enfin broyé.

Les caractéristiques de la silice précipitée S1 ainsi préparée sont rassemblées dans le tableau 1.

EXEMPLE 2

Dans un réacteur en acier inoxydable, d'un volume de 2000 litres, muni d'un système d'agitation par hélices, d'un système d'introduction des réactifs et d'un système de chauffage par double enveloppe, on introduit :

- 67 litres d'une solution de silicate de sodium de rapport molaire $SiO_2/Na_2O$ égal à 3,6 ayant une concentration exprimée en silice de 136 g/l et étant à une température de 65 °C,
- 113 litres d'une solution aqueuse contenant 4,5 kg de $Na_2SO_4$ (électrolyte).

La concentration en silicate exprimée en $SiO_2$ et celle en électrolyte dans le pied de cuve initial sont donc respectivement de 50,6 g/l et de 25,0 g/l. Le mélange obtenu est porté à une température de 92 °C tout en le maintenant sous agitation. L'ensemble de la réaction est effectué à 92 °C.

Dans le pied de cuve ainsi formé et maintenu sous agitation, on introduit d'abord, pendant 5 mn, une solution d'acide sulfurique, de concentration égale à 80 g/l, à un débit de 7,6 l/mn ; à l'issue de cette addition, le taux de neutralisation du pied de cuve est de 73 %, c'est-à-dire que 73 % de la quantité de $Na_2O$ présente dans le pied de cuve initial sont neutralisés.

On introduit ensuite simultanément, pendant 75 mn, dans le milieu réactionnel :

- une solution de silicate de sodium telle que décrite ci-dessus (concentration exprimée en silice de 136 g/l), à une débit de 12 l/mn, et
- une solution d'acide sulfurique telle que décrite ci-dessus (concentration de 80 g/l) à un débit régulé tel que le pH du milieu réactionnel soit égal à 8,9 pendant cette addition simultanée.

On arrête ensuite l'introduction de la solution de silicate de sodium mais on poursuit l'addition de la solution d'acide sulfurique, à un débit de 7,6 l/mn, jusqu'à ce que le pH du milieu réactionnel soit égal à 7,2.

On arrête alors l'introduction de la solution d'acide sulfurique et on laisse mûrir le milieu réactionnel pendant 15 mn, au pH de 7,2 (sous agitation, à 92 °C).

On introduit ensuite de nouveau une solution d'acide sulfurique telle que décrite ci-dessus (concentration de 80 g/l), à un débit de 7,6 l/mn, jusqu'à ce que le pH du milieu réactionnel soit égal à 4,2.

On arrête alors l'introduction de la solution d'acide sulfurique et on laisse mûrir le milieu réactionnel pendant 15 mn, au pH de 4,2 (sous agitation, à 92 °C).

On obtient ainsi une bouillie de silice précipitée qui est filtrée et lavée au moyen d'un filtre rotatif sous vide.

Le gâteau de silice obtenu est ensuite fluidifié par simple action mécanique. Après cette opération de délitage, la bouillie résultante est atomisée au moyen d'un atomiseur à turbines ; le produit séché est enfin broyé.

Les caractéristiques de la silice précipitée S2 ainsi préparée sont rassemblées dans le tableau 1.

EXEMPLE 3

Dans un réacteur en acier inoxydable, d'un volume de 2000 litres, muni d'un système d'agitation par hélices, d'un système d'introduction des réactifs et d'un système de chauffage par double enveloppe, on introduit:

- 115 litres d'une solution de silicate de sodium de rapport molaire $SiO_2/Na_2O$ égal à 3,6 ayant une concentration exprimée en silice de 136 g/l et étant à une température de 65 °C,

- 85 litres d'une solution aqueuse contenant 4,0 kg de $Na_2SO_4$ (électrolyte).

La concentration en silicate exprimée en $SiO_2$ et celle en électrolyte dans le pied de cuve initial sont donc respectivement de 78,2 g/l et de 20,0 g/l. Le mélange obtenu est porté à une température de 90 °C tout en le maintenant sous agitation. L'ensemble de la réaction est effectué à 90 °C.
Dans le pied de cuve ainsi formé et maintenu sous agitation, on introduit d'abord, pendant 7 mn, une solution d'acide sulfurique, de concentration égale à 80 g/l, à un débit de 7,6 l/mn ; à l'issue de cette addition, le taux de neutralisation du pied de cuve est de 60 %, c'est-à-dire que 60 % de la quantité de $Na_2O$ présente dans le pied de cuve initial sont neutralisés.
On introduit ensuite simultanément, pendant 60 mn, dans le milieu réactionnel :

- une solution de silicate de sodium telle que décrite ci-dessus (concentration exprimée en silice de 136 g/l), à une débit de 12 l/mn, et
- une solution d'acide sulfurique telle que décrite ci-dessus (concentration de 80 g/l) à un débit régulé tel que le pH du milieu réactionnel soit égal à 9,2 pendant cette addition simultanée.

On arrête ensuite l'introduction de la solution de silicate de sodium mais on poursuit l'addition de la solution d'acide sulfurique, à un débit de 7,6 l/mn, jusqu'à ce que le pH du milieu réactionnel soit égal à 7,5.
On arrête alors l'introduction de la solution d'acide sulfurique et on laisse mûrir le milieu réactionnel pendant 15 mn, au pH de 7,5 (sous agitation, à 92 °C).
On introduit ensuite de nouveau une solution d'acide sulfurique telle que décrite ci-dessus (concentration de 80 g/l), à un débit de 7,6 l/mn, jusqu'à ce que le pH du milieu réactionnel soit égal à 4,2.
On arrête alors l'introduction de la solution d'acide sulfurique et on laisse mûrir le milieu réactionnel pendant 15 mn, au pH de 4,2 (sous agitation, à 92 °C).
On obtient ainsi une bouillie de silice précipitée qui est filtrée et lavée au moyen d'un filtre rotatif sous vide.
Le gâteau de silice obtenu est ensuite fluidifié par simple action mécanique. Après cette opération de délitage, la bouillie résultante est atomisée au moyen d'un atomiseur à turbines ; le produit séché est enfin broyé.
Les caractéristiques de la silice précipitée S3 ainsi préparée sont rassemblées dans le tableau 1.

EXEMPLE 4

Dans un réacteur en acier inoxydable, d'un volume de 2000 litres, muni d'un système d'agitation par hélices, d'un système d'introduction des réactifs et d'un système de chauffage par double enveloppe, on introduit:

- 117 litres d'une solution de silicate de sodium de rapport molaire $SiO_2/Na_2O$ égal à 3,6 ayant une concentration exprimée en silice de 136 g/l et étant à une température de 65 °C,
- 80 litres d'une solution aqueuse contenant 4,0 kg de $Na_2SO_4$ (électrolyte).

La concentration en silicate exprimée en $SiO_2$ et celle en électrolyte dans le pied de cuve initial sont donc respectivement de 80,7 g/l et de 20,3 g/l. Le mélange obtenu est porté à une température de 92 °C tout en le maintenant sous agitation. L'ensemble de la réaction est effectué à 92 °C.
Dans le pied de cuve ainsi formé et maintenu sous agitation, on introduit d'abord, pendant 7 mn, une solution d'acide sulfurique, de concentration égale à 80 g/l, à un débit de 8,4 l/mn ; à l'issue de cette addition, le taux de neutralisation du pied de cuve est de 65 %, c'est-à-dire que 65 % de la quantité de $Na_2O$ présente dans le pied de cuve initial sont neutralisés.
On introduit ensuite simultanément, pendant 60 mn, dans le milieu réactionnel :

- une solution de silicate de sodium telle que décrite ci-dessus (concentration exprimée en silice de 136 g/l), à une débit de 13,2 l/mn, et
- une solution d'acide sulfurique telle que décrite ci-dessus (concentration de 80 g/l) à un débit régulé tel que le pH du milieu réactionnel soit égal à 9,2 pendant cette addition simultanée.

On arrête ensuite l'introduction de la solution de silicate de sodium mais on poursuit l'addition de la solution d'acide sulfurique, à un débit de 7,6 l/mn, jusqu'à ce que le pH du milieu réactionnel soit égal à 7,5.
On arrête alors l'introduction de la solution d'acide sulfurique et on laisse mûrir le milieu réactionnel pendant 15 mn, au pH de 7,5 (sous agitation, à 92 °C).
On introduit ensuite de nouveau une solution d'acide sulfurique telle que décrite ci-dessus (concentration de 80 g/l), à un débit de 7,6 l/mn, jusqu'à ce que le pH du milieu réactionnel soit égal à 4,2.

On arrête alors l'introduction de la solution d'acide sulfurique et on laisse mûrir le milieu réactionnel pendant 15 mn, au pH de 4,2 (sous agitation, à 92 °C).

On obtient ainsi une bouillie de silice précipitée qui est filtrée et lavée au moyen d'un filtre rotatif sous vide.

Le gâteau de silice obtenu est ensuite fluidifié par simple action mécanique. Après cette opération de délitage, la bouillie résultante est atomisée au moyen d'un atomiseur à turbines ; le produit séché est enfin broyé.

Les caractéristiques de la silice précipitée S4 ainsi préparée sont rassemblées dans le tableau 1.

TABLEAU 1

|  | S1 | S2 | S3 | S4 |
|---|---|---|---|---|
| surface spécifique BET ($m^2$/g) | 35 | 23 | 54 | 29 |
| surface spécifique CTAB ($m^2$/g) | 24 | 22 | 28 | 19 |
| RDA | 135 | 140 | 120 | 147 |
| indice de réfraction | 1,446 | 1,442 | 1,444 | 1,444 |
| transmission à cet indice (%) | 83 | 83 | 78 | 88 |
| prise d'huile DOP (ml/100g) | 98 | 74 | 105 | 95 |
| taille moyenne en poids $D_{50}$ ($\mu$m) | 5,4 | 8,0 | 5,0 | 4,2 |
| pH | 6,2 | 6,3 | 6,4 | 6,2 |
| compatibilité AF (%) | 56 | 58 | 55 | 61 |

**Revendications**

1. Utilisation d'une silice présentant

   i) une surface spécifique BET comprise entre 20 et 75 $m^2$/g,
   ii) une surface spécifique CTAB comprise entre 16 et 45 $m^2$/g,
   iii) une abrasivité RDA comprise entre 120 et 160,
   iv) un indice de réfraction compris entre 1,435 et 1,450,
   v) une prise d'huile DOP comprise entre 70 et 105 ml/100g,
   vi) une compatibilité avec les composés organiques aminés, notamment avec les amines fluorées, d'au moins 50%.

   comme agent abrasif dans une composition dentrifice comprenant un composé organique aminé.

2. Utilisation selon la revendication 1 **caractérisée en ce qu'**elle présente une surface spécifique BET comprise entre 35 et 64 $m^2$/g.

3. Utilisation selon la revendication 2 **caractérisée en ce qu'**elle présente une surface spécifique BET comprise entre 45 et 59 $m^2$/g.

4. Utilisation selon l'une des revendications 1 à 3 **caractérisée en ce qu'**elle présente une surface spécifique CTAB comprise entre 20 et 40 $m^2$/g.

5. Utilisation selon l'une des revendications 1 à 4 **caractérisée en ce qu'**elle présente une abrasivité RDA comprise entre 125 et 145.

**6.** Utilisation selon l'une des revendications 1 à 5 **caractérisée en ce qu'**elle présente un indice de réfraction compris entre 1,438 et 1,446.

**7.** Utilisation selon l'une des revendications 1 à 6 **caractérisée en ce qu'**elle présente une prise d'huile DOP comprise entre 80 et 105 ml/100g.

**8.** Utilisation selon l'une des revendications 1 à 7 **caractérisée en ce qu'**elle présente une compatibilité avec les composés organiques aminés, notamment avec les amines fluorées, d'au moins 55 %.

**9.** Utilisation selon l'une des revendications 1 à 8 **caractérisée en ce qu'**elle présente une transmission supérieure à 70 %.

**10.** Utilisation selon l'une des revendications 1 à 9 **caractérisée en ce qu'**elle présente une taille moyenne en poids $D_{50}$ des particules comprise entre 4 et 20 $\mu$m.

**11.** Utilisation selon l'une des revendications 1 à 10 **caractérisée en ce qu'**elle possède un pH compris entre 6,2 et 7,4.

**12.** Utilisation selon l'une des revendications 1 à 11 **caractérisée en ce que** la différence entre la surface spécifique BET et la surface spécifique CTAB est d'au plus 35 $m^2/g$, en particulier d'au plus 25 $m^2/g$.

**13.** Utilisation selon l'une des revendications 1 à 12 **caractérisée en ce qu'**il s'agit d'une silice précipitée.

**14.** Utilisation selon la revendication 12, **caractérisée en ce que** ladite silice est obtenue par réaction d'un silicate de métal alcalin M avec un agent acidifiant ce par quoi l'on obtient une suspension de silice précipitée, puis la séparation et le séchage de cette suspension, la précipitation étant réalisée selon les étapes successives suivantes :

> (i) on forme un pied de cuve initial comportant une partie de la quantité totale du silicate engagé dans la réaction et au moins un électrolyte, la concentration en silicate exprimée en $SiO_2$ et la concentration en électrolyte dans ledit pied de cuve initial étant comprises respectivement entre 35 et 100 g/l et entre 10 et 40 g/l,
> (ii) on ajoute de l'agent acidifiant audit pied de cuve initial jusqu'à ce que 50 à 85 % de la quantité de $M_2O$ présente dans ledit pied de cuve initial soient neutralisés,
> (iii) on ajoute au milieu réactionnel simultanément de l'agent acidifiant et la quantité restante de silicate, le pH du milieu réactionnel étant maintenu entre 8,6 et 9,6 pendant cette étape (iii).
> (iv) on arrête l'addition de silicate et on poursuit l'addition d'agent acidifiant dans le milieu réactionnel jusqu'à l'obtention d'une valeur de pH dudit milieu comprise entre 7,0 et 8,0,
> (v) on effectue ensuite un premier mûrissement du milieu réactionnel,
> (vi) on ajoute au milieu réactionnel de l'agent acidifiant jusqu'à l'obtention d'une valeur de pH dudit milieu comprise entre 3,7 et 4,6,
> (vii) on effectue enfin un second mûrissement du milieu réactionnel.

**15.** Utilisation selon la revendication 14 **caractérisée en ce que**, pendant l'étape (iii), le pH du milieu réactionnel est maintenu entre 9,0 et 9,4.

**16.** Utilisation selon l'une des revendications 14 et 15 **caractérisée en ce que**, dans l'étape (iv), on ajoute l'agent acidifiant jusqu'à l'obtention d'une valeur de pH du milieu réactionnel comprise entre 7,3 et 7,8.

**17.** Utilisation selon l'une des revendications 14 à 16 **caractérisée en ce que** les durées des étapes (v) et (vii) sont chacune comprises entre 5 et 30 minutes.

**18.** Utilisation selon l'une des revendications 14 à 17 **caractérisée en ce que** la température du milieu réactionnel est maintenue à une température sensiblement constante, comprise entre 75 et 98°C, au cours des étapes (i) à (vii).

**19.** Utilisation selon l'une des revendications 14 à 18 **caractérisée en ce que** ledit séchage est effectué par atomisation.

**20.** Utilisation selon l'une des revendications 14 à 19 **caractérisée en ce que**, à l'issue du séchage, le produit obtenu est broyé.

**Claims**

1. Use of a silica having

   (i) a BET specific surface area of between 20 and 75 m$^2$/g,
   (ii) a CTAB specific surface area of between 16 and 45 m$^2$/g,
   (iii) an RDA abrasiveness of between 120 and 160,
   (iv) a refractive index of between 1.435 and 1.450,
   (v) a DOP oil uptake of between 70 and 150 ml/100g, and
   (vi) a degree of compatibility with organic amino compounds, in particular with fluorinated amines, of at least 50%.

   as an abrasive agent in a dentifrice composition containing an organic amino compound.

2. Use according to claim 1 **characterised in that** has a BET specific surface area of between 35 and 64 m$^2$/g.

3. Use according to claim 2 **characterised in that** it has a BET specific surface area of between 45 and 59 m$^2$/g.

4. Use accrording to one of claims 1 to 3 **characterised in that** it has a CTAB specific surface area of between 20 and 40 m$^2$/g.

5. Use according to one of claims 1 to 4 **characterised in that** it has an RDA abrasiveness of between 125 and 145.

6. Use according to one of claims 1 to 5 **characterised in that** it has a refractive index of between 1.438 and 1.446.

7. Use according to one of claims 1 to 6 **characterised in that** it has a DOP oil uptake of between 80 and 105ml/100g.

8. Use according to one of claims 1 to 7 **characterised in that** it has a degree of compatibility with organic amino compounds, in particular fluorinated amines, of at least 56%.

9. Use according to one of claims 1 to 8 **characterised in that** it has a level of transmission of higher than 70%.

10. Use according to one claims 1 to 9 **characterised in that** it has a mean dimension by weight $D_{50}$ of the particles of between 4 and 20 $\mu$m.

11. Use according to one of claims 1 to 10 **characterised in that** it has a pH-value of between 6.2 and 7.4.

12. Use according to one of claims 1 to 11 **characterised in that** the difference between the BET specific surface area and the CTAB specific surface area is at most 35 m$^2$/g. In particular at most 25 m$^2$/g.

13. Use according to one of claims 1 to 12 **characterised in that** it is a precipitated silica.

14. Use according to claim 13, **characterized in that** the said silica is obtained by reaction of a silicate of alkali metal M with an acidifying agent, thereby obtaining a precipitated silica suspension, then separation and drying of said suspension, the precipitation operation being effected according to the following successive steps:

    (i) an initial sediment is formed comprising a part of the total amount of the silicate involved in the reaction and at least one electrolyte, the concentration of silicate expressed as SiO2 and the concentration of electrolyte in said initial sediment being respectively between 35 and 100 g/l and between 10 and 40 g/l,
    (ii) acidifying agent is added to said initial sediment until 50 to 85% of the amount of $M_2O$ present in said initial sediment is neutralised,
    (iii) acidifying agent and the remaining amount of silicate are simultaneously added to the reaction medium, the pH-value of the reaction medium being maintained at between 8.6 and 9.6 during step (iii),
    (iv) the addition of silicate is stropped and the addition of acidifying agent to the reaction medium is continued until a ph-value of said medium of between 7.0 and 8.0 is attained,
    (v) a first maturing of the reaction medium is effected,
    (vi) acidifying agent is added to the reaction medium until a ph-value of said medium of between 3.7 and 4.6 is attained, and
    (vii) a second maturing of the reaction medium is effected.

**15.** Use according to claim 14 **characterised in that**, during step (iii), the ph-value of the reaction medium is maintained between 9.0 and 9.4.

**16.** Use according to one of claims 14 and 15 **characterised in that**, step (iv), the acidifying agent is added until a pH-value of the reaction medium of between 7.3 and 7.8 is attained.

**17.** Use according to one of claims 14 to 16 **characterised in that** the durations of steps (v) and (vii) are each between 5 and 30 minutes.

**18.** Use according to one of claims 14 to 17 **characterised in that** the temperature of the reaction medium is maintained at a substantially constant temperature of between 75 and 98°C in the course of steps (i) to (vii).

**19.** Use according to one of claims 14 to 18 **characterised in that** said drying operation is effected by atomisation.

**20.** Use according to one of claims 14 to 19 **characterised In that**, at the end of the drying operation, the product obtained is crushed.

**Patentansprüche**

**1.** Verwendung eines Siliciumdioxides, aufweisend:

i) eine spezifische Oberfläche BET zwischen einschließlich 20 $m^2/g$ und 75 $m^2/g$,
ii) eine spezifische Oberfläche CTAB zwischen einschließlich 16 $m^2/g$ und 45 $m^2/g$,
iii) eine Abrasivität RDA zwischen einschließlich 120 und 160,
iv) einen Brechungsindex zwischen einschließlich 1,435 und 1,450,
v) eine Ölaufnahme DOP zwischen einschließlich 70 ml/100 g und 105 ml/100 g,
vi) eine Kompatibilität mit organischen Aminverbindungen, insbesondere mit fluorierten Aminen, von mindestens 50 %,

als abrasives Mittel in einer Zahnpastazusammensetzung, umfassend eine organische Aminverbindung.

**2.** Verwendung nach Anspruch 1, **dadurch gekennzeichnet, dass** es eine spezifische Oberfläche BET zwischen einschließlich 35 $m^2/g$ und 64 $m^2/g$ aufweist.

**3.** Verwendung nach Anspruch 2, **dadurch gekennzeichnet, dass** es eine spezifische Oberfläche BET zwischen einschließlich 45 $m^2/g$ und 59 $m^2/g$ aufweist.

**4.** Verwendung nach einem der Ansprüche 1 bis 3, **dadurch gekennzeichnet, dass** es eine spezifische Oberfläche CTAB zwischen einschließlich 20 $m^2/g$ und 40 $m^2/g$ aufweist.

**5.** Verwendung nach einem der Ansprüche 1 bis 4, **dadurch gekennzeichnet, dass** es eine Abrasivität RDA zwischen einschließlich 125 und 145 aufweist.

**6.** Verwendung nach einem der Ansprüche 1 bis 5, **dadurch gekennzeichnet, dass** es einen Brechungsindex zwischen einschließlich 1,438 und 1,446 aufweist.

**7.** Verwendung nach einem der Ansprüche 1 bis 6, **dadurch gekennzeichnet, dass** es eine Ölaufnahme DOP zwischen einschließlich 80 ml/100 g und 105 ml/100 g aufweist.

**8.** Verwendung nach einem der Ansprüche 1 bis 7, **dadurch gekennzeichnet, dass** es eine Kompatibilität mit organischen Aminverbindungen, insbesondere mit fluorierten Aminen, von mindestens 55 % aufweist.

**9.** Verwendung nach einem der Ansprüche 1 bis 8, **dadurch gekennzeichnet, dass** es eine Transmission von höher als 70 % aufweist.

**10.** Verwendung nach einem der Ansprüche 1 bis 9, **dadurch gekennzeichnet, dass** es eine mittlere Größe $D_{50}$ in Gewicht der Teilchen zwischen einschließlich 4 $\mu$m und 20 $\mu$m aufweist.

**11.** Verwendung nach einem der Ansprüche 1 bis 10, **dadurch gekennzeichnet, dass** es einen pH-Wert zwischen einschließlich 6,2 und 7,4 besitzt.

**12.** Verwendung nach einem der Ansprüche 1 bis 11, **dadurch gekennzeichnet, dass** die Differenz zwischen der spezifischen Oberfläche BET und der spezifischen Oberfläche CTAB höchstens 35 $m^2$/g und insbesondere höchstens 25 $m^2$/g beträgt.

**13.** Verwendung nach einem der Ansprüche 1 bis 12, **dadurch gekennzeichnet, dass** es sich um ein gefälltes Siliciumdioxid handelt.

**14.** Verwendung nach Anspruch 12, **dadurch gekennzeichnet, dass** das genannte Siliciumdioxid durch Reaktion eines Silicates eines Alkalimetalls M mit einem säurebildenden Mittel in der Weise erhalten wird, dass man eine Suspension von gefälltem Siliciumdioxid erhält und anschließend die Trennung und die Trocknung dieser Suspension durchführt, wobei die Fällung nach den folgenden schrittweisen Stufen realisiert wird:

i) man bildet einen Ausgangsansatz, der einen Teil der Gesamtmenge des bei der Reaktion eingesetzten Silicates und mindestens einen Elektrolyten umfaßt, wobei die Konzentration an Silicat, ausgedrückt in $SiO_2$, und die Konzentration an Elektrolyt in dem genannten Ausgangsansatz jeweils zwischen einschließlich 35 g/l und 100 g/l und zwischen 10 g/l und 40 g/l liegen,

ii) man gibt das säurebildende Mittel zu dem genannten Ausgangsansatz, bis 50 % bis 85 % der in dem genannten Ausgangsansatz anwesenden Menge von $M_2O$ neutralisiert sind,

iii) man gibt gleichzeitig das säurebildende Mittel und die restliche Menge des Silicates zu dem Reaktionsmedium, wobei der pH-Wert des Reaktionsmediums während dieser Stufe (iii) zwischen 8,6 und 9,6 gehalten wird,

iv) man beendet die Zugabe des Silicates und setzt die Zugabe des säurebildenden Mittels zu dem Reaktionsmedium fort, bis man einen pH-Wert des genannten Mediums zwischen einschließlich 7,0 und 8,0 erhält,

v) man führt anschließend eine erste Reifung des Reaktionsmediums durch,

vi) man gibt das säurebildende Mittel zu dem Reaktionsmedium, bis man einen pH-Wert des genannten Mediums zwischen einschließlich 3,7 und 4,6 erhält,

vii) man führt schließlich eine zweite Reifung des Reaktionsmediums durch.

**15.** Verwendung nach Anspruch 14, **dadurch gekennzeichnet, dass** während der Stufe (iii) der pH-Wert des Reaktionsmediums zwischen 9,0 und 9,4 gehalten wird.

**16.** Verwendung nach einem der Ansprüche 14 und 15, **dadurch gekennzeichnet, dass** man in der Stufe (iv) das säurebildende Mittel zugibt, bis man einen pH-Wert des Reaktionsmediums zwischen einschließlich 7,3 und 7,8 erhält.

**17.** Verwendung nach einem der Ansprüche 14 bis 16, **dadurch gekennzeichnet, dass** die Dauer der Stufen (v) und (vii) jeweils zwischen einschließlich 5 und 30 Minuten beträgt.

**18.** Verwendung nach einem der Ansprüche 14 bis 17, **dadurch gekennzeichnet, dass** die Temperatur des Reaktionsmediums im Verlauf der Stufen (i) bis (vii) auf einer nahezu konstanten Temperatur zwischen einschließlich 75°C und 98°C gehalten wird.

**19.** Verwendung nach einem der Ansprüche 14 bis 18, **dadurch gekennzeichnet, dass** die genannte Trocknung mittels Versprühen durchgeführt wird.

**20.** Verwendung nach einem der Ansprüche 14 bis 19, **dadurch gekennzeichnet, dass** nach Abschluß der Trocknung das erhaltene Produkt zerkleinert wird.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 139754 A

- EP 236070 A

**Littérature non-brevet citée dans la description**

- **BRUNAUER-EMMET-TELLER.** *The Journal of the American Chemical Society,* Février 1938, vol. 60, 309

- **J.J. HEFFERREN.** *Journal of Dental Research,* 1976, vol. 55 (4), 563